# EUROPEAN PATENT APPLICATION

(11) **EP 3 103 508 A1**
(43) Date of publication of application: **14.12.2016**
(21) Application number: 16174035.2
(22) Date of filing: 10.06.2016
(51) Int. Cl.: A61N 1/05, A61N 1/36, A61N 1/372, A61N 1/378

(54) **EMBEDDED FIXATION DEVICES OR LEADS**

(30) Priority: 11.06.2015 US 201562174047 P
(71) Applicant: Micron Devices, LLC, Miami Beach, FL 33139 (US)
(72) Inventor: Perryman, Laura Tyler, Miami Beach, FL 33139 (US); Andresen, Chad David, Miami Beach, FL 33141 (US); Al-Kaisy, Adnan, Miami Beach, FL 33139 (US)
(74) Representative: Richardt Patentanwälte PartG mbB

(57) **Abstract**

A wireless neural stimulator device can include: a housing configured to house: a first antenna configured to receive, from a second antenna and through electrical radiative coupling, an input signal containing electrical energy, the second antenna being physically separate from the wireless neural stimulator device; one or more flexible circuits electrically connected to the first antenna, the flexible circuits configured to create the one or more electrical pulses suitable to be applied at the electrodes using the electrical energy contained in the input signal and supply the one or more electrical pulses to one or more electrodes; one or more electrodes disposed on the housing, the one or more electrodes being coupled to the flexible circuits to receive the one or more electrical pulses supplied by the one or more flexible circuits; one or more fixation features disposed on the housing and configured to fixate the device to tissue.

## Description

### TECHNICAL FIELD

This description is related to implantable devices or leads.

### BACKGROUND

A variety of therapeutic intra-body electrical stimulation techniques are utilized to treat neuropathic conditions that utilize a subcutaneous battery operated implantable pulse generator (IPG) connected to one or more implantable wired leads with connectors on the end. These devices have numerous failure modes, including mechanical dislodgement due to motion, acceleration and impingement of the lead electrode assembly, infection, and uncomfortable irritation. These device configurations include cylindrical percutaneous leads with small diameter and a number of cylindrical electrodes. The majority of adverse events associated with administration of therapies where such leads are utilized in human tissue locations are related to the ability to fixate the device or lead body in place without migration away from the desired target. This migration results in a loss of therapy or measurement, revision surgery, replacement of the device, and an ongoing cost burden on the health care system.

### Summary

In one aspect, some implementations provide a wireless neural stimulator device that includes: a housing configured to house: a first antenna configured to receive, from a second antenna and through electrical radiative coupling, an input signal containing electrical energy, the second antenna being physically separate from the wireless neural stimulator device; one or more flexible circuits electrically connected to the first antenna, the flexible circuits configured to create the one or more electrical pulses suitable to be applied at the electrodes using the electrical energy contained in the input signal and supply the one or more electrical pulses to one or more electrodes; one or more electrodes disposed on the housing, the one or more electrodes being coupled to the flexible circuits to receive the one or more electrical pulses supplied by the one or more flexible circuits; one or more fixation features disposed on the housing and configured to fixate the device to tissue.

Implementations may include one or more of the following features.

The one or more fixation features may include a cuff formed from a porous material that promotes tissue in-growth. The one or more fixation features may include a surface feature configured to promote tissue adhesion. The surface feature may include an altered surface area that is increased relative to the surface area of other portions of the housing. The surface feature may include spirals or dimples. The one or more fixation features may include a tine band. The tine band may include a hub with a central opening and one or more tines extending radially from the hub and forming an acute angle with respect to a central axis passing through a center of the opening of the hub. The tines may be resilient. The electrodes may be spaced from the first antenna such that, when the device is implanted in a patient in a position to stimulate a dorsal root ganglion or exiting nerves of the spinal cord, the first antenna is surrounded by fatty tissue. The electrodes and the first antenna may be spaced such that, when the device is implanted in a patient in a position to stimulate the sacral nerve, the first antenna is substantially parallel to a surface of a back of the patient. The electrodes may be configured for monopolar stimulation. The electrodes may include multiple electrodes disposed at a distal end of the housing and a remote electrode disposed at the proximal end of the housing, the remote electrode configured as an anode for monopolar stimulation.

### BRIEF DESCRIPTION OF THE DRAWINGS

FIG. 1 depicts a high-level diagram of an example of a stimulation system that includes a device to be fixed in tissue.
FIG. 2 depicts a detailed diagram of an example of a stimulation system that includes a device to be fixed in tissue.
FIG. 3 is a circuit diagram showing an example of an implantable stimulator device to be fixed in tissue.
FIG. 4 is a circuit diagram of another example of an implantable stimulator device to be fixed in tissue.
FIG. 5 illustrates an example of a cylindrical stimulator device to be fixed in tissue.
FIG. 6 illustrates an implementation of a stimulator for subcutaneous placement to be fixed in tissue.
FIG. 7 illustrates an implementation of a stimulator device for dorsal-root ganglion (DRG) placement to be fixed in tissue.
FIG. 8A illustrates an implementation of a stimulator device for placement at the sacral nerve through the sacral foramen to be fixed in tissue.
FIG. 8B illustrates a stimulator placed at the sacral nerve through the sacral foramen to be fixed in tissue.
FIG. 9A illustrates an example of a method for implanting a stimulator into a person to stimulate a nerve to be fixed in tissue.
FIG. 9B illustrates the stimulator in place near a nerve after being implanted into the human body for stimulation applications.
FIG. 10 is a diagram illustrating an example of a tine band that may be used in some implementations as a fixation feature.
FIG. 11 illustrates an implementation of a stimulator device that includes tine bands as fixation features.
FIG. 12A illustrates an implementation of a stimulator device without tissue-ingrowth promoting features while implanted in tissue.
FIG. 12B illustrates an implementation of a stimulator device with tissue-ingrowth surface treatment while implanted in tissue.
FIG. 12C illustrates an implementation of a stimulator device with tissue-ingrowth cuff while implanted in tissue.

Like reference symbols in the various drawings indicate like elements.

### DETAILED DESCRIPTION

In various implementations, systems and methods are disclosed for applying one or more electrical impulses to targeted excitable tissue, such as nerves, for treating pain, such as chronic pain, or other modalities, such as inflammation, arthritis, sleep apnea, seizures, incontinence, pain associated with cancer, incontinence, problems of movement initiation and control, involuntary movements, vascular insufficiency, heart arrhythmias, angina, peripheral vascular disease, gastrointestinal disorders, obesity, diabetes, craniofacial pain, such as migraines or cluster headaches, and other disorders. In certain embodiments, a device may be used to send electrical energy to targeted nerve tissue by using, for example, remote radio frequency (RF) energy, without cables or inductive coupling to power a passive implanted wireless stimulator device. The targeted nerves can include, but are not limited to, the spinal cord and surrounding areas, including the spinothalamic tracts, the dorsal horn, dorsal root ganglion, the exiting nerve roots, nerve ganglions, the dorsal column fibers, sacral nerve roots, and the peripheral nerve bundles leaving the dorsal column and brain, such as the vagus, occipital, trigeminal, hypoglossal, sacral, coccygeal nerves and the like, as well as any cranial nerves, abdominal, thoracic, or trigeminal ganglia nerves, nerve bundles of the cerebral cortex, deep brain and any sensory or motor nerves.

A stimulation system can include an implantable stimulator device that includes an enclosure housing one or more conductive antennas, and internal circuitry for frequency waveform and electrical energy rectification, and includes one or more electrodes. The system may further comprise an external controller and antenna for transmitting radio frequency or microwave energy from an external source to the implantable stimulator device with neither cables nor inductive coupling to provide power.

In various implementations, the stimulator device is powered wirelessly (and therefore does not require a wired connection) and contains the circuitry necessary to receive the pulse instructions from a source external to the body. For example, various embodiments employ internal dipole (or other) antenna configuration(s) to receive RF power through electrical radiative coupling. This allows such devices to produce electrical currents capable of stimulating nerve bundles without a physical connection to an implantable pulse generator (IPG) or use of an inductive coil.

Further descriptions of exemplary wireless systems for providing neural stimulation to a patient can be found in commonly-assigned, co-pending applications PCT/US2012/23029 filed January 28, 2011, PCT/US2012/32200 filed April 11, 2011, PCT/US2012/48903, filed January 28, 2011, PCT/US2012/50633, filed August 12, 2011, PCT/US2012/55746, filed September 15, 2011, and U.S. Application 14/590,524 filed January 6, 2015, the complete disclosures of which are incorporated by reference.

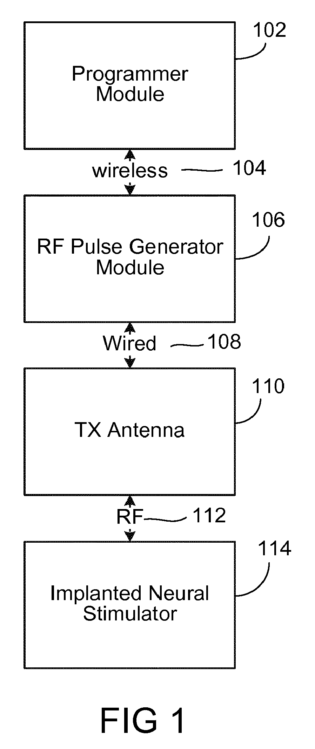

FIG. 1 depicts a high-level diagram of an example of a wireless stimulation system. The wireless stimulation system may include four major components, namely, a programmer module **102,** a RF pulse generator module **106,** a transmit (TX) antenna **110** (for example, a patch antenna, slot antenna, or a dipole antenna), and an implanted wireless stimulator device **114.** The programmer module **102** may be a computer device, such as a smart phone, running a software application that supports a wireless connection **104,** such as Bluetooth®. The application can enable the user to view the system status and diagnostics, change various parameters, increase/decrease the desired stimulus amplitude of the electrode pulses, and adjust feedback sensitivity of the RF pulse generator module **106,** among other functions.

The RF pulse generator module **106** may include communication electronics that support the wireless connection **104,** the stimulation circuitry, and the battery to power the generator electronics. In some implementations, the RF pulse generator module **106** includes the TX antenna embedded into its packaging form factor while, in other implementations, the TX antenna is connected to the RF pulse generator module **106** through a wired connection **108** or a wireless connection (not shown). The TX antenna **110** may be coupled directly to tissue to create an electric field that powers the implanted wireless stimulator device **114.** The TX antenna **110** communicates with the implanted wireless stimulator device **114** through an RF interface. For instance, the TX antenna **110** radiates an RF transmission signal that is modulated and encoded by the RF pulse generator module **110.** The implanted wireless stimulator device of module 114 contains one or more antennas, such as dipole antenna(s), to receive and transmit through RF interface **112.** In particular, the coupling mechanism between antenna **110** and the one or more antennas on the implanted wireless stimulation device of module **114** utilizes electrical radiative coupling and not inductive coupling. In other words, the coupling is through an electric field rather than a magnetic field.

Through this electrical radiative coupling, the TX antenna **110** can provide an input signal to the implanted wireless stimulator device **114.** This input signal contains energy and may contain information encoding stimulus waveforms to be applied at the electrodes of the implanted wireless stimulator device **114.** In some implementations, the power level of this input signal directly determines an applied amplitude (for example, power, current, or voltage) of the one or more electrical pulses created using the electrical energy contained in the input signal. Within the implanted wireless stimulator device **114** are components for demodulating the RF transmission signal, and electrodes to deliver the stimulation to surrounding neuronal tissue.

The RF pulse generator module **106** can be implanted subcutaneously, or it can be worn external to the body. When external to the body, the RF generator module **106** can be incorporated into a belt or harness design to allow for electric radiative coupling through the skin and underlying tissue to transfer power and/or control parameters to the implanted wireless stimulator device **114.** In either event, receiver circuit(s) internal to the wireless stimulator device **114** (or cylindrical wireless implantable stimulator device 1400 shown in FIGS. 14A and 14B, helical wireless implantable stimulator device 1900 shown in FIGS. 19A to 19C) can capture the energy radiated by the TX antenna **110** and convert this energy to an electrical waveform. The receiver circuit(s) may further modify the waveform to create an electrical pulse suitable for the stimulation of neural tissue.

In some implementations, the RF pulse generator module **106** can remotely control the stimulus parameters (that is, the parameters of the electrical pulses applied to the neural tissue) and monitor feedback from the wireless stimulator device **114** based on RF signals received from the implanted wireless stimulator device **114.** A feedback detection algorithm implemented by the RF pulse generator module **106** can monitor data sent wirelessly from the implanted wireless stimulator device **114,** including information about the energy that the implanted wireless stimulator device **114** is receiving from the RF pulse generator and information about the stimulus waveform being delivered to the electrode pads. In order to provide an effective therapy for a given medical condition, the system can be tuned to provide the optimal amount of excitation or inhibition to the nerve fibers by electrical stimulation. A closed loop feedback control method can be used in which the output signals from the implanted wireless stimulator device **114** are monitored and used to determine the appropriate level of neural stimulation current for maintaining effective neuronal activation, or, in some cases, the patient can manually adjust the output signals in an open loop control method.

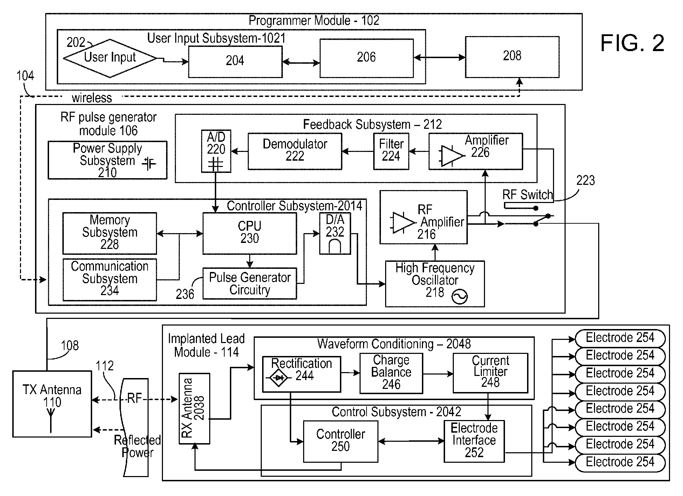

FIG. **2** depicts a detailed diagram of an example of the wireless stimulation system. As depicted, the programming module **102** may comprise user input system **202** and communication subsystem **208.** The user input system **221** may allow various parameter settings to be adjusted (in some cases, in an open loop fashion) by the user in the form of instruction sets. The communication subsystem **208** may transmit these instruction sets (and other information) via the wireless connection **104,** such as Bluetooth or Wi-Fi, to the RF pulse generator module **106,** as well as receive data from module **106.**

For instance, the programmer module **102,** which can be utilized for multiple users, such as a patient's control unit or clinician's programmer unit, can be used to send stimulation parameters to the RF pulse generator module **106.** The stimulation parameters that can be controlled may include pulse amplitude, pulse frequency, and pulse width in the ranges shown in Table **1.** In this context the term pulse refers to the phase of the waveform that directly produces stimulation of the tissue; the parameters of the charge-balancing phase (described below) can similarly be controlled. The patient and/or the clinician can also optionally control overall duration and pattern of treatment.

**Stimulation Parameter Table 1**

| | |
|---|---|
| Pulse Amplitude: | 0 to 20 mA |
| Pulse Frequency: | 0 to 20000 Hz |
| Pulse Width: | 0 to 2 ms |

The RF pulse generator module **106** may be initially programmed to meet the specific parameter settings for each individual patient during the initial implantation procedure. Because medical conditions or the body itself can change over time, the ability to re-adjust the parameter settings may be beneficial to ensure ongoing efficacy of the neural modulation therapy.

The programmer module **102** may be functionally a smart device and associated application. The smart device hardware may include a CPU **206** and be used as a vehicle to handle touchscreen input on a graphical user interface (GUI) **204,** for processing and storing data.

The RF pulse generator module **106** may be connected via wired connection **108** to an external TX antenna **110.** Alternatively, both the antenna and the RF pulse generator are located subcutaneously (not shown).

The signals sent by RF pulse generator module **106** to the implanted wireless stimulator device **114** may include both power and parameter-setting attributes in regards to stimulus waveform, amplitude, pulse width, and frequency. The RF pulse generator module **106** can also function as a wireless receiving unit that receives feedback signals from the implanted wireless stimulator device **114.** To that end, the RF pulse generator module **106** may contain microelectronics or other circuitry to handle the generation of the signals transmitted to the device **114** as well as handle feedback signals, such as those from the stimulator device **114.** For example, the RF pulse generator module **106** may comprise controller subsystem **214,** high-frequency oscillator **218,** RF amplifier **216,** a RF switch, and a feedback subsystem **212.**

The controller subsystem **214** may include a CPU **230** to handle data processing, a memory subsystem **228** such as a local memory, communication subsystem **234** to communicate with programmer module **102** (including receiving stimulation parameters from programmer module), pulse generator circuitry **236,** and digital/analog (D/A) converters **232.**

The controller subsystem **214** may be used by the patient and/or the clinician to control the stimulation parameter settings (for example, by controlling the parameters of the signal sent from RF pulse generator module **106** to the stimulator device **114**). These parameter settings can affect, for example, the power, current level, or shape of the one or more electrical pulses. The programming of the stimulation parameters can be performed using the programming module **102,** as described above, to set the repetition rate, pulse width, amplitude, and waveform that will be transmitted by RF energy to the receiving (RX) antenna **238,** typically a dipole antenna (although other types may be used), in the implanted wireless stimulation device **214.** The clinician may have the option of locking and/or hiding certain settings within the programmer interface, thus limiting the patient's ability to view or adjust certain parameters because adjustment of certain parameters may require detailed medical knowledge of neurophysiology, neuroanatomy, protocols for neural modulation, and safety limits of electrical stimulation.

The controller subsystem **214** may store received parameter settings in the local memory subsystem **228,** until the parameter settings are modified by new input data received from the programming module **102.** The CPU **206** may use the parameters stored in the local memory to control the pulse generator circuitry **236** to generate a stimulus waveform that is modulated by a high frequency oscillator **218** in the range from **300** MHz to **8** GHz (preferably between about 700 MHz and 5.8 GHz and more preferably between about 800 MHz and 1.3 GHz). The resulting RF signal may then be amplified by RF amplifier **226** and then sent through an RF switch **223** to the TX antenna **110** to reach through depths of tissue to the RX antenna **238.**

In some implementations, the RF signal sent by TX antenna **110** may simply be a power transmission signal used by the wireless stimulation device module **114** to generate electric pulses. In other implementations, a telemetry signal may also be transmitted to the wireless stimulator device **114** to send instructions about the various operations of the wireless stimulator device **114.** The telemetry signal may be sent by the modulation of the carrier signal (through the skin if external, or through other body tissues if the pulse generator module **106** is implanted subcutaneously). The telemetry signal is used to modulate the carrier signal (a high frequency signal) that is coupled onto the implanted antenna(s) **238** and does not interfere with the input received on the same stimulator device to power the device. In one embodiment the telemetry signal and powering signal are combined into one signal, where the RF telemetry signal is used to modulate the RF powering signal, and thus the wireless stimulation device is powered directly by the received telemetry signal; separate subsystems in the wireless stimulation device harness the power contained in the signal and interpret the data content of the signal.

The RF switch 223 may be a multipurpose device such as a dual directional coupler, which passes the relatively high amplitude, extremely short duration RF pulse to the TX antenna **110** with minimal insertion loss while simultaneously providing two low-level outputs to feedback subsystem **212;** one output delivers a forward power signal to the feedback subsystem **212,** where the forward power signal is an attenuated version of the RF pulse sent to the TX antenna **110,** and the other output delivers a reverse power signal to a different port of the feedback subsystem **212,** where reverse power is an attenuated version of the reflected RF energy from the TX Antenna **110.**

During the on-cycle time (when an RF signal is being transmitted to wireless stimulator device **114**), the RF switch **223** is set to send the forward power signal to feedback subsystem. During the off-cycle time (when an RF signal is not being transmitted to the wireless stimulator device **114**), the RF switch **223** can change to a receiving mode in which the reflected RF energy and/or RF signals from the wireless stimulator device **114** are received to be analyzed in the feedback subsystem **212.**

The feedback subsystem **212** of the RF pulse generator module **106** may include reception circuitry to receive and extract telemetry or other feedback signals from the wireless stimulator device **114** and/or reflected RF energy from the signal sent by TX antenna **110.** The feedback subsystem may include an amplifier **226,** a filter **224,** a demodulator **222,** and an A/D converter **220.**

The feedback subsystem **212** receives the forward power signal and converts this high-frequency AC signal to a DC level that can be sampled and sent to the controller subsystem **214.** In this way the characteristics of the generated RF pulse can be compared to a reference signal within the controller subsystem **214.** If a disparity (error) exists in any parameter, the controller subsystem **214** can adjust the output to the RF pulse generator **106.** The nature of the adjustment can be, for example, proportional to the computed error. The controller subsystem **214** can incorporate additional inputs and limits on its adjustment scheme such as the signal amplitude of the reverse power and any predetermined maximum or minimum values for various pulse parameters.

The reverse power signal can be used to detect fault conditions in the RF-power delivery system. In an ideal condition, when TX antenna **110** has perfectly matched impedance to the tissue that it contacts, the electromagnetic waves generated from the RF pulse generator **106** pass unimpeded from the TX antenna **110** into the body tissue. However, in real-world applications a large degree of variability may exist in the body types of users, types of clothing worn, and positioning of the antenna **110** relative to the body surface. Since the impedance of the antenna **110** depends on the relative permittivity of the underlying tissue and any intervening materials, and also depends on the overall separation distance of the antenna from the skin, in any given application there can be an impedance mismatch at the interface of the TX antenna **110** with the body surface. When such a mismatch occurs, the electromagnetic waves sent from the RF pulse generator **106** are partially reflected at this interface, and this reflected energy propagates backward through the antenna feed.

The dual directional coupler RF switch **223** may prevent the reflected RF energy propagating back into the amplifier **226,** and may attenuate this reflected RF signal and send the attenuated signal as the reverse power signal to the feedback subsystem **212.** The feedback subsystem **212** can convert this high-frequency AC signal to a DC level that can be sampled and sent to the controller subsystem **214.** The controller subsystem **214** can then calculate the ratio of the amplitude of the reverse power signal to the amplitude of the forward power signal. The ratio of the amplitude of reverse power signal to the amplitude level of forward power may indicate severity of the impedance mismatch.

In order to sense impedance mismatch conditions, the controller subsystem **214** can measure the reflected-power ratio in real time, and according to preset thresholds for this measurement, the controller subsystem **214** can modify the level of RF power generated by the RF pulse generator **106.** For example, for a moderate degree of reflected power the course of action can be for the controller subsystem **214** to increase the amplitude of RF power sent to the TX antenna **110,** as would be needed to compensate for slightly non-optimum but acceptable TX antenna coupling to the body. For higher ratios of reflected power, the course of action can be to prevent operation of the RF pulse generator **106** and set a fault code to indicate that the TX antenna **110** has little or no coupling with the body. This type of reflected-power fault condition can also be generated by a poor or broken connection to the TX antenna. In either case, it may be desirable to stop RF transmission when the reflected-power ratio is above a defined threshold, because internally reflected power can result in unwanted heating of internal components, and this fault condition means the system cannot deliver sufficient power to the implanted wireless stimulation device and thus cannot deliver therapy to the user.

The controller **242** of the wireless stimulator device **114** may transmit informational signals, such as a telemetry signal, through the antenna **238** to communicate with the RF pulse generator module **106** during its receive cycle. For example, the telemetry signal from the wireless stimulator device **114** may be coupled to the modulated signal on the dipole antenna(s) **238,** during the on and off state of the transistor circuit to enable or disable a waveform that produces the corresponding RF bursts necessary to transmit to the external (or remotely implanted) pulse generator module **106.** The antenna(s) **238** may be connected to electrodes **254** in contact with tissue to provide a return path for the transmitted signal. An A/D (not shown) converter can be used to transfer stored data to a serialized pattern that can be transmitted on the pulse-modulated signal from the internal antenna(s) **238** of the wireless stimulator device **114.**

A telemetry signal from the implanted wireless stimulator device **114** may include stimulus parameters such as the power or the amplitude of the current that is delivered to the tissue from the electrodes. The feedback signal can be transmitted to the RF pulse generator module **116** to indicate the strength of the stimulus at the nerve bundle by means of coupling the signal to the implanted RX antenna **238,** which radiates the telemetry signal to the external (or remotely implanted) RF pulse generator module **106.** The feedback signal can include either or both an analog and digital telemetry pulse modulated carrier signal. Data such as stimulation pulse parameters and measured characteristics of stimulator performance can be stored in an internal memory device within the implanted stimulator device **114,** and sent on the telemetry signal. The frequency of the carrier signal may be in the range of at **300** MHz to **8** GHz (preferably between about 700 MHz and 5.8 GHz and more preferably between about 800 MHz and 1.3 GHz).

In the feedback subsystem **212,** the telemetry signal can be down modulated using demodulator **222** and digitized by being processed through an analog to digital (A/D) converter **220.** The digital telemetry signal may then be routed to a CPU **230** with embedded code, with the option to reprogram, to translate the signal into a corresponding current measurement in the tissue based on the amplitude of the received signal. The CPU **230** of the controller subsystem **214** can compare the reported stimulus parameters to those held in local memory **228** to verify the wireless stimulator device **114** delivered the specified stimuli to tissue. For example, if the wireless stimulation device reports a lower current than was specified, the power level from the RF pulse generator module **106** can be increased so that the implanted wireless stimulator device **114** will have more available power for stimulation. The implanted wireless stimulator device **114** can generate telemetry data in real time, for example, at a rate of **8** Kbits per second. All feedback data received from the implanted stimulator device **114** can be logged against time and sampled to be stored for retrieval to a remote monitoring system accessible by the health care professional for trending and statistical correlations.

The sequence of remotely programmable RF signals received by the internal antenna(s) **238** may be conditioned into waveforms that are controlled within the implantable wireless stimulator device **114** by the control subsystem **242** and routed to the appropriate electrodes **254** that are placed in proximity to the tissue to be stimulated. For instance, the RF signal transmitted from the RF pulse generator module **106** may be received by RX antenna **238** and processed by circuitry, such as waveform conditioning circuitry **240,** within the implanted wireless stimulator device **114** to be converted into electrical pulses applied to the electrodes **254** through electrode interface **252.** In some implementations, the implanted wireless stimulator device **114** contains between two to sixteen electrodes **254.**

The waveform conditioning circuitry **240** may include a rectifier **244,** which rectifies the signal received by the RX antenna **238.** The rectified signal may be fed to the controller **242** for receiving encoded instructions from the RF pulse generator module **106.** The rectifier signal may also be fed to a charge balance component **246** that is configured to create one or more electrical pulses based such that the one or more electrical pulses result in a substantially zero net charge at the one or more electrodes (that is, the pulses are charge balanced). The charge-balanced pulses are passed through the current limiter **248** to the electrode interface **252,** which applies the pulses to the electrodes **254** as appropriate.

The current limiter **248** insures the current level of the pulses applied to the electrodes **254** is not above a threshold current level. In some implementations, an amplitude (for example, current level, voltage level, or power level) of the received RF pulse directly determines the amplitude of the stimulus. In this case, it may be particularly beneficial to include current limiter **248** to prevent excessive current or charge being delivered through the electrodes, although current limiter **248** may be used in other implementations where this is not the case. Generally, for a given electrode having several square millimeters surface area, it is the charge per phase that should be limited for safety (where the charge delivered by a stimulus phase is the integral of the current). But, in some cases, the limit can instead be placed on the current, where the maximum current multiplied by the maximum possible pulse duration is less than or equal to the maximum safe charge. More generally, the limiter **248** acts as a charge limiter that limits a characteristic (for example, current or duration) of the electrical pulses so that the charge per phase remains below a threshold level (typically, a safe-charge limit).

In the event the implanted wireless stimulator device **114** receives a "strong" pulse of RF power sufficient to generate a stimulus that would exceed the predetermined safe-charge limit, the current limiter **248** can automatically limit or "clip" the stimulus phase to maintain the total charge of the phase within the safety limit. The current limiter **248** may be a passive current limiting component that cuts the signal to the electrodes **254** once the safe current limit (the threshold current level) is reached. Alternatively, or additionally, the current limiter **248** may communicate with the electrode interface **252** to turn off all electrodes **254** to prevent tissue damaging current levels.

A clipping event may trigger a current limiter feedback control mode. The action of clipping may cause the controller to send a threshold power data signal to the pulse generator **106.** The feedback subsystem **212** detects the threshold power signal and demodulates the signal into data that is communicated to the controller subsystem **214.** The controller subsystem **214** algorithms may act on this current-limiting condition by specifically reducing the RF power generated by the RF pulse generator, or cutting the power completely. In this way, the pulse generator **106** can reduce the RF power delivered to the body if the implanted wireless stimulator device **114** reports it is receiving excess RF power.

The controller **250** of the stimulator **205** may communicate with the electrode interface **252** to control various aspects of the electrode setup and pulses applied to the electrodes **254.** The electrode interface **252** may act as a multiplex and control the polarity and switching of each of the electrodes **254.** For instance, in some implementations, the wireless stimulator **106** has multiple electrodes **254** in contact with tissue, and for a given stimulus the RF pulse generator module **106** can arbitrarily assign one or more electrodes to **1**) act as a stimulating electrode, **2**) act as a return electrode, or **3**) be inactive by communication of assignment sent wirelessly with the parameter instructions, which the controller **250** uses to set electrode interface **252** as appropriate. It may be physiologically advantageous to assign, for example, one or two electrodes as stimulating electrodes and to assign all remaining electrodes as return electrodes.

Also, in some implementations, for a given stimulus pulse, the controller **250** may control the electrode interface **252** to divide the current arbitrarily (or according to instructions from pulse generator module **106**) among the designated stimulating electrodes. This control over electrode assignment and current control can be advantageous because in practice the electrodes **254** may be spatially distributed along various neural structures, and through strategic selection of the stimulating electrode location and the proportion of current specified for each location, the aggregate current distribution in tissue can be modified to selectively activate specific neural targets. This strategy of current steering can improve the therapeutic effect for the patient.

In another implementation, the time course of stimuli may be arbitrarily manipulated. A given stimulus waveform may be initiated at a time T_start and terminated at a time T_final, and this time course may be synchronized across all stimulating and return electrodes; further, the frequency of repetition of this stimulus cycle may be synchronous for all the electrodes. However, controller **250,** on its own or in response to instructions from pulse generator **106,** can control electrode interface **252** to designate one or more subsets of electrodes to deliver stimulus waveforms with non-synchronous start and stop times, and the frequency of repetition of each stimulus cycle can be arbitrarily and independently specified.

For example, a stimulator having eight electrodes may be configured to have a subset of five electrodes, called set A, and a subset of three electrodes, called set B. Set A might be configured to use two of its electrodes as stimulating electrodes, with the remainder being return electrodes. Set B might be configured to have just one stimulating electrode. The controller **250** could then specify that set A deliver a stimulus phase with **3** mA current for a duration of **200** us followed by a **400** us charge-balancing phase. This stimulus cycle could be specified to repeat at a rate of **60** cycles per second. Then, for set B, the controller **250** could specify a stimulus phase with 1 mA current for duration of 500 us followed by a 800 us charge-balancing phase. The repetition rate for the set-B stimulus cycle can be set independently of set A, say for example it could be specified at **25** cycles per second. Or, if the controller **250** was configured to match the repetition rate for set B to that of set A, for such a case the controller **250** can specify the relative start times of the stimulus cycles to be coincident in time or to be arbitrarily offset from one another by some delay interval.

In some implementations, the controller **250** can arbitrarily shape the stimulus waveform amplitude, and may do so in response to instructions from pulse generator **106.** The stimulus phase may be delivered by a constant-current source or a constant-voltage source, and this type of control may generate characteristic waveforms that are static, e.g. a constant-current source generates a characteristic rectangular pulse in which the current waveform has a very steep rise, a constant amplitude for the duration of the stimulus, and then a very steep return to baseline. Alternatively, or additionally, the controller **250** can increase or decrease the level of current at any time during the stimulus phase and/or during the charge-balancing phase. Thus, in some implementations, the controller **250** can deliver arbitrarily shaped stimulus waveforms such as a triangular pulse, sinusoidal pulse, or Gaussian pulse for example. Similarly, the charge-balancing phase can be arbitrarily amplitude-shaped, and similarly an anodic pulse (prior to the stimulus phase) may also be amplitude-shaped.

As described above, the wireless stimulator device **114** may include a charge-balancing component **246.** Generally, for constant current stimulation pulses, pulses should be charge balanced by having the amount of cathodic current should equal the amount of anodic current, which is typically called biphasic stimulation. Charge density is the amount of current times the duration it is applied, and is typically expressed in the units uC/cm². In order to avoid the irreversible electrochemical reactions such as pH change, electrode dissolution as well as tissue destruction, no net charge should appear at the electrode-electrolyte interface, and it is generally acceptable to have a charge density less than 30 uC/cm². Biphasic stimulating current pulses ensure that no net charge appears at the electrode after each stimulation cycle and the electrochemical processes are balanced to prevent net dc currents. The wireless stimulator device **114** may be designed to ensure that the resulting stimulus waveform has a net zero charge. Charge balanced stimuli are thought to have minimal damaging effects on tissue by reducing or eliminating electrochemical reaction products created at the electrode-tissue interface.

A stimulus pulse may have a negative- voltage or current, called the cathodic phase of the waveform. Stimulating electrodes may have both cathodic and anodic phases at different times during the stimulus cycle. An electrode that delivers a negative current with sufficient amplitude to stimulate adjacent neural tissue is called a "stimulating electrode." During the stimulus phase the stimulating electrode acts as a current sink. One or more additional electrodes act as a current source and these electrodes are called "return electrodes." Return electrodes are placed elsewhere in the tissue at some distance from the stimulating electrodes. When a typical negative stimulus phase is delivered to tissue at the stimulating electrode, the return electrode has a positive stimulus phase. During the subsequent charge-balancing phase, the polarities of each electrode are reversed.

In some implementations, the charge balance component **246** uses a blocking capacitor(s) placed electrically in series with the stimulating electrodes and body tissue, between the point of stimulus generation within the stimulator circuitry and the point of stimulus delivery to tissue. In this manner, a resistor-capacitor (RC) network may be formed. In a multi-electrode stimulator, one charge-balance capacitor(s) may be used for each electrode or a centralized capacitor(s) may be used within the stimulator circuitry prior to the point of electrode selection. The RC network can block direct current (DC), however it can also prevent low-frequency alternating current (AC) from passing to the tissue. The frequency below which the series RC network essentially blocks signals is commonly referred to as the cutoff frequency, and in one embodiment the design of the stimulator system may ensure the cutoff frequency is not above the fundamental frequency of the stimulus waveform. In this embodiment as disclosed herein, the wireless stimulator may have a charge-balance capacitor with a value chosen according to the measured series resistance of the electrodes and the tissue environment in which the stimulator is implanted. By selecting a specific capacitance value the cutoff frequency of the RC network in this embodiment is at or below the fundamental frequency of the stimulus pulse.

In other implementations, the cutoff frequency may be chosen to be at or above the fundamental frequency of the stimulus, and in this scenario the stimulus waveform created prior to the charge-balance capacitor, called the drive waveform, may be designed to be non-stationary, where the envelope of the drive waveform is varied during the duration of the drive pulse. For example, in one embodiment, the initial amplitude of the drive waveform is set at an initial amplitude Vi, and the amplitude is increased during the duration of the pulse until it reaches a final value k*Vi. By changing the amplitude of the drive waveform over time, the shape of the stimulus waveform passed through the charge-balance capacitor is also modified. The shape of the stimulus waveform may be modified in this fashion to create a physiologically advantageous stimulus.

In some implementations, the wireless stimulator device **114** may create a drive-waveform envelope that follows the envelope of the RF pulse received by the receiving dipole antenna(s) **238.** In this case, the RF pulse generator module **106** can directly control the envelope of the drive waveform within the wireless stimulator device **114,** and thus no energy storage may be required inside the stimulator itself. In this implementation, the stimulator circuitry may modify the envelope of the drive waveform or may pass it directly to the charge-balance capacitor and/or electrode-selection stage.

In some implementations, the implanted wireless stimulator device **114** may deliver a single-phase drive waveform to the charge balance capacitor or it may deliver multiphase drive waveforms. In the case of a single-phase drive waveform, for example, a negative-going rectangular pulse, this pulse comprises the physiological stimulus phase, and the charge-balance capacitor is polarized (charged) during this phase. After the drive pulse is completed, the charge balancing function is performed solely by the passive discharge of the charge-balance capacitor, where is dissipates its charge through the tissue in an opposite polarity relative to the preceding stimulus. In one implementation, a resistor within the stimulator facilitates the discharge of the charge-balance capacitor. In some implementations, using a passive discharge phase, the capacitor may allow virtually complete discharge prior to the onset of the subsequent stimulus pulse.

In the case of multiphase drive waveforms the wireless stimulator may perform internal switching to pass negative-going or positive-going pulses (phases) to the charge-balance capacitor. These pulses may be delivered in any sequence and with varying amplitudes and waveform shapes to achieve a desired physiological effect. For example, the stimulus phase may be followed by an actively driven charge-balancing phase, and/or the stimulus phase may be preceded by an opposite phase. Preceding the stimulus with an opposite-polarity phase, for example, can have the advantage of reducing the amplitude of the stimulus phase required to excite tissue.

In some implementations, the amplitude and timing of stimulus and charge-balancing phases is controlled by the amplitude and timing of RF pulses from the RF pulse generator module **106,** and in others this control may be administered internally by circuitry onboard the wireless stimulator device **114,** such as controller **250.** In the case of onboard control, the amplitude and timing may be specified or modified by data commands delivered from the pulse generator module **106.**

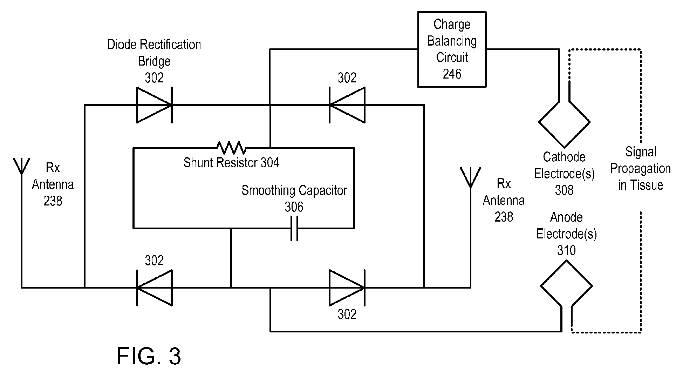

FIG. **3** is a circuit diagram showing an example of a wireless stimulator device **114.** This example contains paired electrodes, comprising cathode electrode(s) **308** and anode electrode(s) **310,** as shown. When energized, the charged electrodes create a volume conduction field of current density within the tissue. In this implementation, the wireless energy is received through a dipole antenna(s) **238.** At least four diodes are connected together to form a full wave bridge rectifier **302** attached to the dipole antenna(s) **238.** Each diode, up to **100** micrometers in length, uses a junction potential to prevent the flow of negative electrical current, from cathode to anode, from passing through the device when said current does not exceed the reverse threshold. For neural stimulation via wireless power, transmitted through tissue, the natural inefficiency of the lossy material may cause a low threshold voltage. In this implementation, a zero biased diode rectifier results in a low output impedance for the device. A resistor **304** and a smoothing capacitor **306** are placed across the output nodes of the bridge rectifier to discharge the electrodes to the ground of the bridge anode. The rectification bridge **302** includes two branches of diode pairs connecting an anode-to-anode and then cathode to cathode. The electrodes **308** and **310** are connected to the output of the charge balancing circuit **246.**

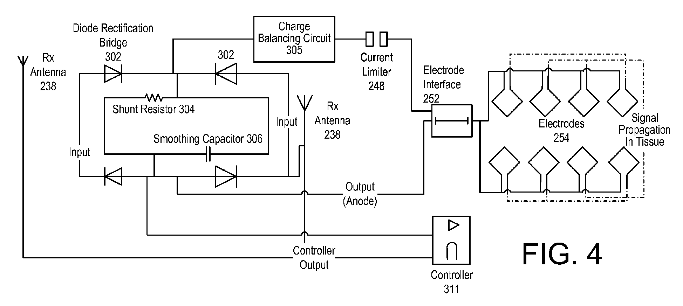

FIG. **4** is a circuit diagram of another example of a wireless stimulator device **114.** The example shown in FIG. **4** includes multiple electrode control and may employ full closed loop control. The wireless stimulation device includes an electrode array **254** in which the polarity of the electrodes can be assigned as cathodic or anodic, and for which the electrodes can be alternatively not powered with any energy. When energized, the charged electrodes create a volume conduction field of current density within the tissue. In this implementation, the wireless energy is received by the device through the dipole antenna(s) **238.** The electrode array **254** is controlled through an on-board controller circuit **242** that sends the appropriate bit information to the electrode interface **252** in order to set the polarity of each electrode in the array, as well as power to each individual electrode. The lack of power to a specific electrode would set that electrode in a functional OFF position. In another implementation (not shown), the amount of current sent to each electrode is also controlled through the controller **242.** The controller current, polarity and power state parameter data, shown as the controller output, is be sent back to the antenna(s) **238** for telemetry transmission back to the pulse generator module **106.** The controller **242** also includes the functionality of current monitoring and sets a bit register counter so that the status of total current drawn can be sent back to the pulse generator module **106.**

At least four diodes can be connected together to form a full wave bridge rectifier **302** attached to the dipole antenna(s) **238.** Each diode, up to **100** micrometers in length, uses a junction potential to prevent the flow of negative electrical current, from cathode to anode, from passing through the device when said current does not exceed the reverse threshold. For neural stimulation via wireless power, transmitted through tissue, the natural inefficiency of the lossy material may cause a low threshold voltage. In this implementation, a zero biased diode rectifier results in a low output impedance for the device. A resistor **404** and a smoothing capacitor **406** are placed across the output nodes of the bridge rectifier to discharge the electrodes to the ground of the bridge anode. The rectification bridge **402** may include two branches of diode pairs connecting an anode-to-anode and then cathode to cathode. The electrode polarity outputs, both cathode **408** and anode **410** are connected to the outputs formed by the bridge connection. Charge balancing circuitry **246** and current limiting circuitry **248** are placed in series with the outputs.

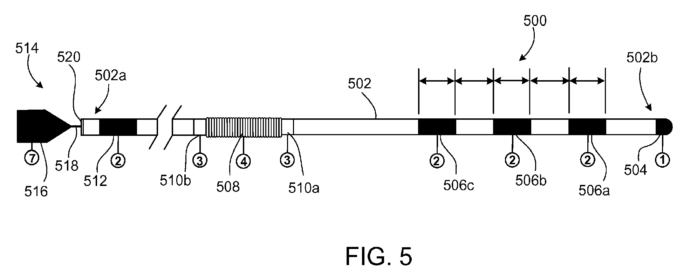

FIG. 5 illustrates an example of a cylindrical implantable wireless neural stimulator device 500. The device 500 may have a diameter between about 0.8 mm and about 1.4 mm and includes a generally cylindrical housing 502 with a distal end 502b and a proximal end 502a.

The distal end 502b terminates in a non-conductive tip 504 that is rounded with, for example, a length of between about 0.5 mm and about 2.0 mm. The distal tip 504 that includes, for example, a smooth finish for navigating the lead through anatomical structures, such as the epidural space.

The distal end 502b includes electrodes 506a, 506b, and 506b. Each electrode 506a, 506b, and 506b can be configured in anode, cathode, or neutral state, as described above. While three electrodes are shown, the distal end 502b may include between two and sixteen electrodes. The electrodes 506a, 506b, and 506b may have a longitudinal length, L, of between about 1.0 mm and about 6.0 and width of between about 0.4 mm and 3.0 mm. The spacing, S, between the electrodes 506a, 506b, and 506b may be between about 1.0 mm and about 6.0 mm. The total electrode surface area may be between about 0.8 mm² and about 60.0 mm². In certain embodiments, 3.0 mm electrode are utilized with 3.0 mm of spacing between each electrode. The length of the housing 502 between the electrode 506a and tip 504 may be, for example, 1.0 mm to 5.0 mm. The electrodes 506a, 506b, and 506b may be made of at least one of: platinum, platinum-iridium, gallium-nitride, titanium-nitride, iridium-oxide, or combinations thereof.

In certain implementations, the proximal end 502a includes an electrode 512 that can be used for monopolar stimulation to provide for precise focal stimulation. This separated electrode 512 is connected to circuitry and acts as a remote anode for indications where the stimulation field at the distal electrode array should be very precise. The remote electrode polarity can be configured between anode, cathode or neutral. The separated electrode may be a length from between 5 cm to 10 cm proximal from the proximal-most electrode. In the case of implementing monopolar stimulation, electrode 512 is set to be an anode; one of the distal electrodes, 506a, 506b, 506c is set to cathode. In systems that involve an IPG, the case acts as an anode.

The proximal end 502a also includes an opening 520 to an inner lumen of the housing 502 that extends longitudinally from the opening 520 to the distal tip 504. A bent tip stylet 514 includes a handle 516 at the proximal end of the stylet 514 and a shaft 518 that can be inserted into the inner lumen and, when fully inserted, terminates at the distal tip 504. The distal end of the shaft is bent to aid a physician in maneuvering the stylet 514 and device 500 within the body of a patient.

The device 500 includes one or more fixation features 508. In general, the fixation features 508 operate to fixate the device 500 to the tissue in which the device 500 is implanted. In some implementations, the fixation feature 508 may be a tissue-ingrowth cuff made of a porous material, such as Dacron or a knitted fiber or metal, that has a lattice structure or chemical composition to promote tissue in-growth. The fixation feature 508 may be a cylinder of such material disposed on the outer surface of the housing 502. These cuffs can range in length from between 1 mm to 10 mm. The cuffs can range in diameter from between 1.1 mm to 1.5 mm so as to fit through standard introducers or needles.

In other implementations, the fixation feature 508 may be a surface treatment of the housing 502 that results in a surface configured to promote adhesion with tissue to form scar tissue. For example, the surface may be treated so that the surface area is increased exponentially through one or more methods of treatment which may include, pre-formed tubing, chemical treatment, mold heating, or post-assembly treatment. This may result in a pivoted surface in which the surface area is expanded significantly to promote tissue fibrosis. The surface feature may include reflowing shapes that may include three-dimensional spirals and dimples. The treatments may be performed on the existing polymer body or can be added as a layer. Surface treatments can range in length from between 1 mm to 10 mm. In the sections of surface treatment, the device diameter may be decreased to a total diameter from between 1.0 mm to 1.5 mm so as to increase surface area. The total diameter is maintained to allow the device to pass through standard introducers or needles.

The device 500 also includes marking bands 510a and 510b to identify the device 500 model, or variant, or for use as fiducial guidance.

In some implementations, the device may be formed from multiple layers. For example, the housing 502 may be formed from a biocompatible compound that elicits minimal scar tissue formation, such as polymethymethacrylate (PMMA), polydimethylsiloxane (PDMS), parylene, polyurethane, Ethylene TetraFluoroEthylene (ETFE), polytetrafluoroethylene (PTFE), or polycarbonate. The durometer of the material utilized for the housing 502 may allow for easy bending and guidance of the stimulator or lead body to targeted tissue. The fixation feature 508 may be formed as a layer of material on the housing 502. These materials may include polymers such as, without limitation, Polyethylene terephthalate (PET) "Dacron™", polymethymethacrylate (PMMA), polydimethylsiloxane (PDMS), parylene, polyurethane, Ethylene TetraFluoroEthylene (ETFE), polytetrafluoroethylene (PTFE), or polycarbonate. The tissue-ingrowth promoting materials may be manufactured in a lattice structure to promote optimal tissue binding. In a lattice structure, the material in a smooth straight form may not promote any tissue ingrowth, for example PTFE which is used because of its non-stick properties. This material in a three-dimensional lattice allows the body's fibrosis-causing building blocks to scar the device around and into the body's tissue. The electrodes 506a, 506b, and 506c may similarly be formed as a metallic layer on housing 502. Another layer of material that may be included contains a small relative permeability and low conductivity metal located above the antennas to allow for optimal coupling with an exterior antenna (not depicted). Yet another layer can comprise a coating of a silicone elastomer to assist in preventing migration of the device 500 by adhering to the surrounding tissue.

Electronics, such as those described above for receiving signals and generated stimulation waveforms, are located within the inner lumen or other area of housing 502. For example, the housing 502 houses a first antenna configured to receive, from a second antenna and through electrical radiative coupling, an input signal containing electrical energy, and one or more flexible circuits electrically connected to the first antenna and configured to create one or more electrical pulses suitable to be applied at the electrodes using the electrical energy contained in the input signal and supply the one or more electrical pulses to the one or more electrodes.

The device 500 can be delivered into any tissue within a subject's body. The delivery can be through a needle, such as, for example, a tuohy needle, no larger than 14 gauge. The device 500 may be delivered to treat a neural tissue. For example, for spinal cord stimulation, needles may be inserted through the outer skin of the body through a small incision in the skin and in between the vertebrae at no more than a 45-degree angle, lateral to the spinous processes off the midline, and the device 500 may be introduced and placed against the dura of the spinal column to lie perpendicularly to the spinal cord. The device 500 can contain extension tubing that terminate just under the entry point of the skin.

Excluding the electrodes, which are coupled to the surrounding tissue, the remaining portions of the device 500 may be insulated from surrounding body tissue partially or totally by an external coating layer of biocompatible dielectric material with a low dielectric constant. Materials with rigidity similar to that of tissue can be used to reduce the risk of migration and the development of fibrous scar tissue. Such fibrous scar tissue can increase electrode-tissue impedance. If the electrode-tissue impedance can be kept low, less energy may be consumed to achieve stimulation of the targeted tissues.

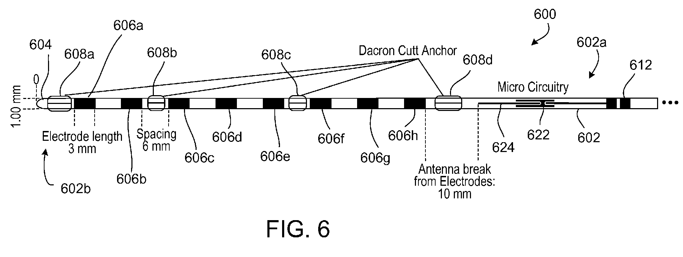

FIG. 6 illustrates an implementation of a wireless neural stimulator 600 for subcutaneous placement. The device 600 is like device 500, but is optimal for placement in and near craniofacial nerves including but not limited to the Trigeminal nerve branches, occiptial nerve, and sphenopalatine ganglion. Other subcutaneous placements for device 600 include the posterior thoracic, anterior thoracic, arms, and legs. The device 600 is 1.0 mm in diameter and includes eight electrodes 606a-606h. The electrodes 606a-606h are each 3 mm in length and have a spacing of 6 mm. A separate marker band 612 is provided as radiopaque markers for easy explantation and location. Four fixation features 608a-608d are formed as Dacron cuffs along the length of the device 600. The receiving antenna 624 and electronics 622 are located in the proximal end 602a, but as distal as possible within 1 cm away from distal-most electrode to decrease the length of the device 600 for optimal subcutaneous placement to match with external antenna without extraneous routing. For example, the distance between the antenna 624 and the first electrode 606h is 10mm. In some embodiments, this distance is between about 10 mm to 50 mm, which allows the contacts to be placed at deeper nerve targets, such as the sphenopalatine ganglion or the trigeminal ganglion but allow the electronics to remain subcutaneous.

In general, the rounded tip 604 can be a non-conductive tip with a length of between 0.5 mm and 2.0 mm, and a smooth finish for navigating the lead through soft tissue space. The device 600 may have between two and sixteen cylindrical electrodes on its distal end with a diameter between about 0.8 mm and about 1.4 mm. The electrodes may have a longitudinal length of between about 1.0 mm and about 6.0 mm from the distal tip toward the proximal tip. The spacing between the electrode contacts may be between about 1.0 mm and about 6.0 mm. The total electrode surface area of the cylindrical wireless lead body may be between about 1.6 mm2 and about 60.0 mm2.

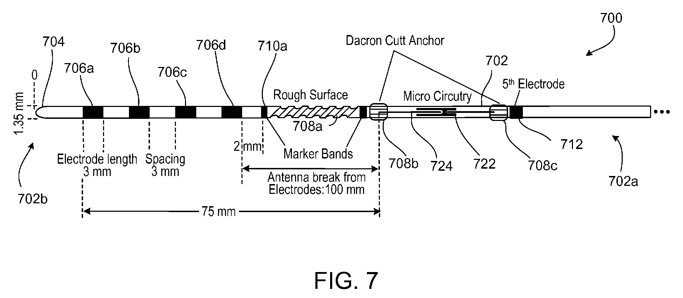

FIG. 7 illustrates an implementation of a wireless neural stimulator device 700 for dorsal-root ganglion (DRG) placement in the spinal cord. The device 600 is like device 500, but is optimal for placement in and near DRGs or the exiting nerves of the spinal cord. The device 600 may be placed to reach a DRG either through Dorsal column, transgrade, or transforaminal methods. The device 700 is 1.35 mm in diameter and includes four electrodes 706a-706d. The electrodes 706a-706d are each 3 mm in length and have a spacing of 3 mm. A separate electrode 712 is provided for monopolar stimulation. The electrode 72 acts as an anode, and is located proximal of the electronics 722 and antenna 724.

There are three fixation features 708a-708c formed along the length of the device 700. Fixation features 708b and 708c are Dacron cuffs. Fixation feature 708a is a surface feature on the housing body to increase surface area and tissue bonding. The feature 708a shown is a spiral pattern formed on the surface of the housing 702.

The electronics 722 and antenna 724 are located in the proximal end 702a at a specific distance, from 1 mm to 10 mm, proximal of the electrodes to allow the length of the antenna to be surrounded by fatty tissue when implanted, rather than bone, for optimal RF coupling. In the example shown, the proximal end of the antenna 724 is located 7 mm from the first electrode 706d.

In some embodiments, marker bands 710a and 710b are implemented to identify the model, or variant, or for use as fiducial guidance.

In general, the rounded tip 704 can be a non-conductive tip with a length of between 0.5 mm and 2.0 mm, and a smooth finish for navigating the lead through the epidural space. The device 700 may have between two and five cylindrical electrodes on its distal end with a diameter between about 0.8 mm and about 1.4 mm. The electrodes may have a longitudinal length of between about 1.0 mm and about 3.0 mm from the distal tip toward the proximal tip. The spacing between the electrode contacts may be between about 1.0 mm and about 3.0 mm. The total electrode surface area may be between about 1.6 mm² and about 24.0 mm².

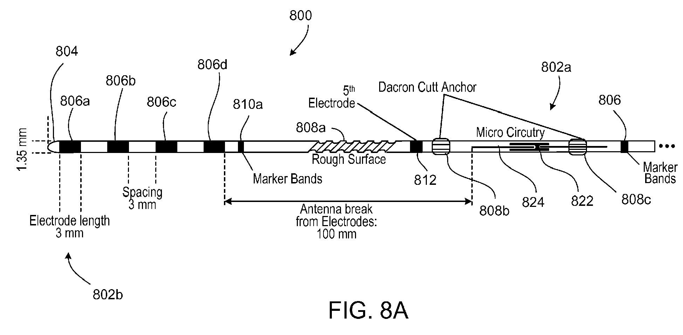

FIG. 8A illustrates an implementation of a wireless neural stimulator device 800 for placement at the sacral nerve through the sacral foramen. The device 800 is like device 500, but is optimal for placement near the sacral nerve in the sacral foramen. The device 800 is 1.35 mm in diameter and includes four electrodes 806a-806d. The electrodes 806a-806d are each 3 mm in length and have a spacing of 3 mm. A separate electrode 812 is provide for monopolar stimulation. The electrode 812 acts as an anode, and is located proximal of the electronics 822 and antenna 824 to allow focal point cathodic stimulation on the sacral nerve.

There are three fixation features 808a-808c formed along the length of the device 800. Fixation features 808b and 808c are Dacron cuffs. Fixation feature 808a is a surface feature on the housing body to increase surface area and tissue bonding. The feature 808a shown is a spiral pattern formed on the surface of the housing 802. In the sacral foramen, this surface feature would promote tissue ingrowth before the device 800 exits the foramen for anchoring.

The electronics 822 and antenna 824 are located in the proximal end 802a at a specific distance, from about 50 mm to 100 mm, proximal of the electrodes to allow there to be enough space without critical electronics as the device 800 makes the up to 90° bend cranially when exiting the sacral foramen. This allows the receiving electronics 822 and antenna 824 to be in a parallel plane with the transmitting electronics that send the input signal. In the example shown, the proximal end of the antenna 824 is located 100 mm from the first electrode 806d.

In some embodiments, marker bands 810a and 810b are implemented to identify the model, or variant, or depth markers, or for use as fiducial guidance.

In general, rounded tip 804 can be a non-conductive tip with a length of between 0.5 mm and 2.0 mm, and a smooth finish for navigating the lead into the sacral nerve space. The device 800 may have between two and five cylindrical electrodes on its distal end with a diameter between about 0.8 mm and about 1.4 mm. The electrodes may have a longitudinal length of between about 1.0 mm and about 3.0 mm from the distal tip toward the proximal tip. The spacing between the electrode contacts may be between about 1.0 mm and about 3.0 mm. The total electrode surface area may be between about 1.6 mm² and about 24.0 mm².

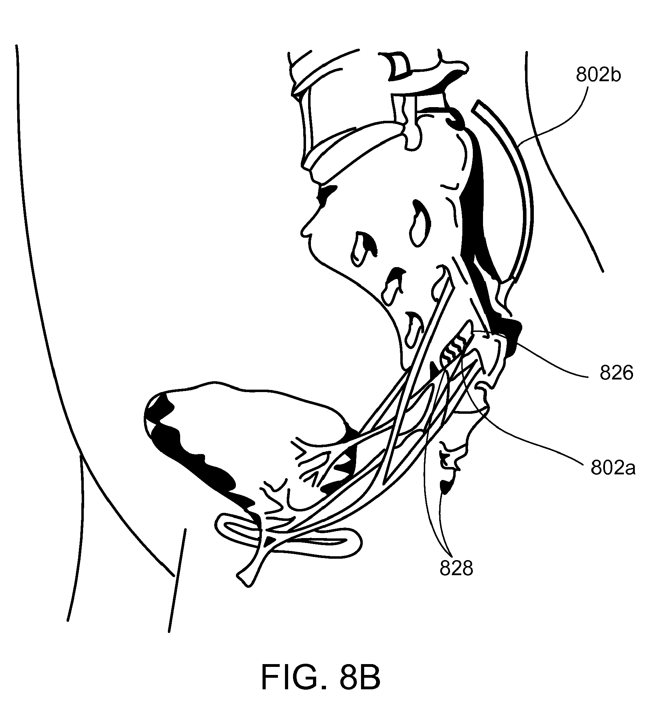

FIG. 8B illustrates the device 800 placed at the sacral nerve through the sacral foramen. As shown, the distal end 802b is passed anteriorly through the sacral foramen 826 such that the electrodes 806a-806d are located near the sacral nerve 828. The proximal end 802a extends out the posterior side of the sacral foramen and extends cranially such that the proximal end 802a, along with electronics 822 and antenna 824, are generally parallel to the surface of the patient's back.

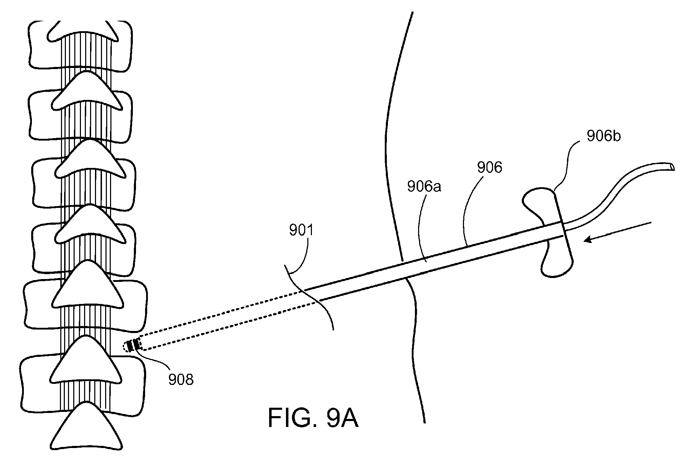

FIG. 9A illustrates an example of a method for implanting a wireless neural stimulator, such as device 700, into a person to stimulate a nerve. In this case, the stimulator is implanted through an introducer cannula 906. The introducer cannula 906 has an elongated, generally tubular sheath section 906a and a handle section 906b. The introducer 906 includes an inner lumen that extends longitudinally from an opening at the handle section 906b, through the handle section 906b and sheath section 906a, to an opening at the end of the sheath section 906a.

Initially, a treating physician makes a small surgical incision 901, for example, less than 2 mm long, on the surface of the patient skin. For example, the surgical incision 901 may be made by cutting the skin using a scalpel and under local anesthesia. Next, the introducer cannula 906 or Tuohy needle (not shown) is inserted through surgical incision 901 underneath the patient's skin and advanced until the distal end of the introducer cannula 906 is near a target site for implantation..

Once the introducer 906 is situated, a stimulator device 908 is inserted through the opening, located at the proximal end of introducer 906, to the inner lumen of the introducer and advanced through the inner lumen until the tip of the wireless neural stimulator device 908 passes through the opening at the end of the sheath section 906a. For example, the clinician may advance the stimulator device 908 through the inner lumen until substantial resistance is met as a result of the tip 908a passing through the opening and contacting tissue. Thereafter, the introducer cannula 906 is withdrawn.

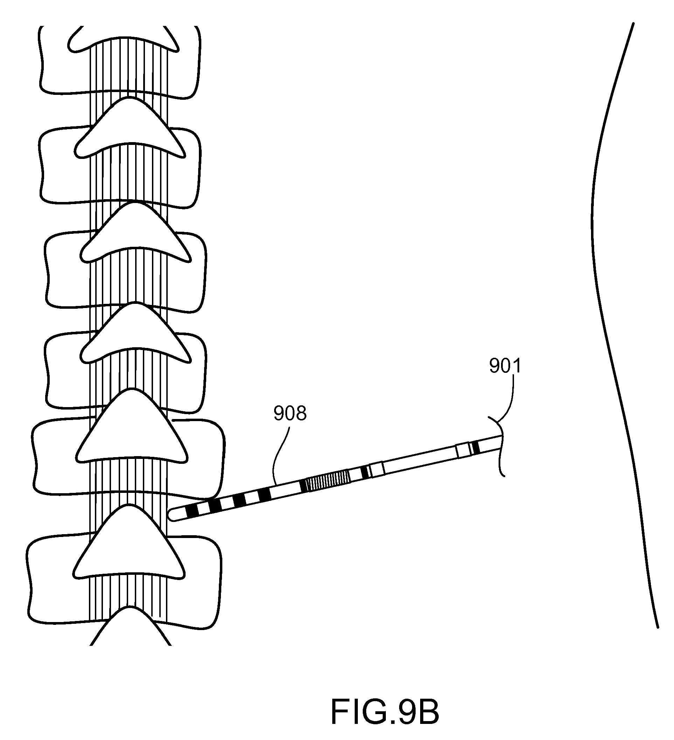

FIG. 9B illustrates the neural stimulator 908 in place near a nerve after being implanted into the human body for stimulation applications. In some cases, after the introducer 906 has been removed, neural stimulator device 908 may be anchored, for example, by suturing to the fascia or layer of "tougher" tissue below the skin, any excess portion of the device 908 extending outside of incision 901 may be trimmed, and the surgical incision 901 may be sealed. In some instances, the proximal end of wireless neural stimulator device 908 may be anchored or sutured to the surrounding tissue near the original site of surgical incision 901. To suture the device 908, the clinician may run the suture stitch directly through its soft plastic body or loop suture around the stimulator device 908 itself noninvasively. The small incision at the skin can be stitched with a suture or sterile strip.

While an anchoring step, such as suturing the device 908 to the fascia, may be employed, such a step may be optional when a fixation feature is included on the device 908. The fixation feature may provide appropriate fixation or anchoring of the device within the tissue medium to prevent migration. In that case, the physician may forego a separate anchoring of the device and, instead, proceed directly to trimming any excess portion of the device (if needed) and closing the surgical incision 901.

In some cases, multiple wireless neural stimulators may be implanted through an extended-width introducer.

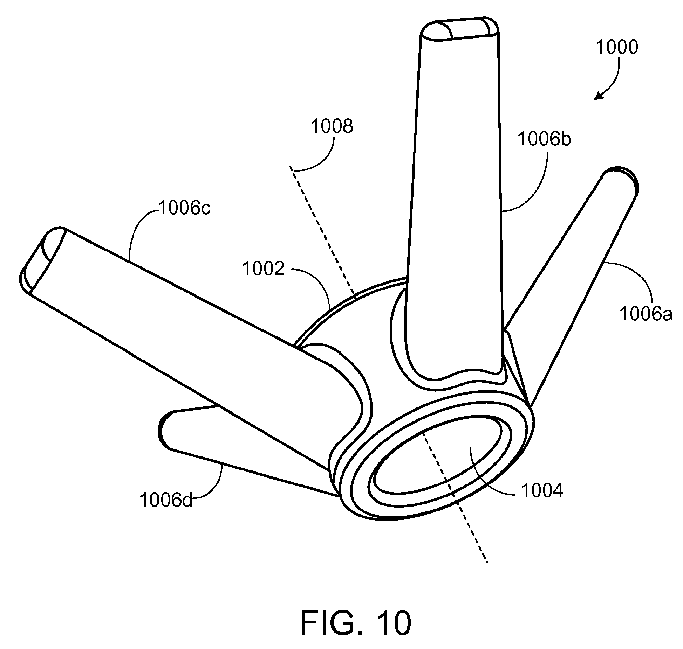

FIG. 10 is a diagram illustrating an example of a tine band 1000 that may be used in some implementations as a fixation feature as an alternative, or in addition to, a surface treatment or cuff. The tine band 1000 includes a cylindrical hub 1002 with a central opening 1004 and multiple tines 1006a-1006d extending radially from the hub. The tines 1006a-1006d extend radially from the hub 1002 such that the tines 1006a-1006d each form an acute angle with respect to a central axis passing through the center of opening 1004. One or more tine bands 1000 may be placed longitudinally along the housing of a neural stimulator. The number of tine-bands may be, for example, from one to eight pieces. Each tine band may include from two to four individual tines.

The tines 1006a-1006d may act to increase total surface area while limiting directional movement. Depending on the orientation, the tines 1006a-1006d prevent proximal or distal movement after placement. In some cases, one or more tine bands may be oriented in the same direction so that all of the tine bands prevent movement in a single direction, ether distal or proximal. In other cases, multiple tine bands may be oriented to prevent both proximal and distal movement.

The tines 1006a-1006d may be formed from a resilient material such that the tines 1006a-1006d may be compressed radially while the neural stimulator is being implanted through an introducer and then expand radially once they exit the introducer. Depending on placement, the tines may expand into tissue as the introducer needle or sheath is pulled out. The tine material may be made of a polymer such as silicone, or polyurethane. The durometer may vary as a function of the needed fixation strength. For example, for stimulators implanted around sensitive tissue areas, a durometer from 70A to 55D may be used.

As an alternative, rather than a tine band 1000 with a hub 1002 that is placed over the stimulator housing, the tines 1006a-1006d may be integrally formed with the stimulator housing.

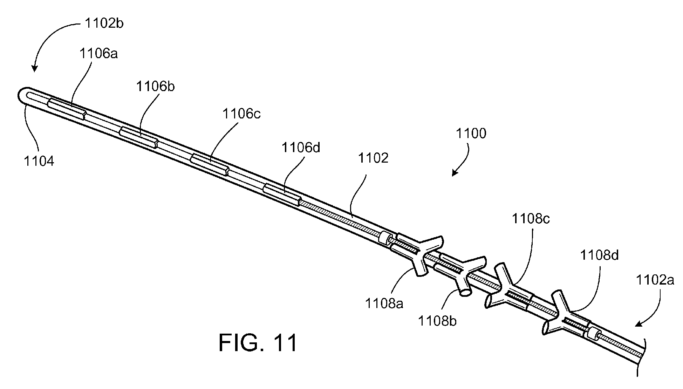

FIG. 11 illustrates an implementation of a wireless neural stimulator device 1100 that includes tine bands 1108a-1108d as fixation features. The device 1100 is like device 500, and includes a housing 1102 with and a proximal end 1102a and a distal end 1102b that terminates in a distal tip 1104. The housing 1102 houses the electronics (not shown) and antenna (not shown). Four electrodes 1106a-1106d are disposed on the housing 1102 at the proximal end 1102b. There are four tine bands 1108a-1108d placed along the length of the device 1100. Tine bands 1108a and 1108b are oriented such that the tines extend at least partially in the proximal direction from the respective hubs and therefore prevent movement in the proximal direction. Tine bands 1108c and 1108d are oriented such that the tines extend at least partially in the distal direction from the respective hubs and therefore prevent movement in the distal direction.

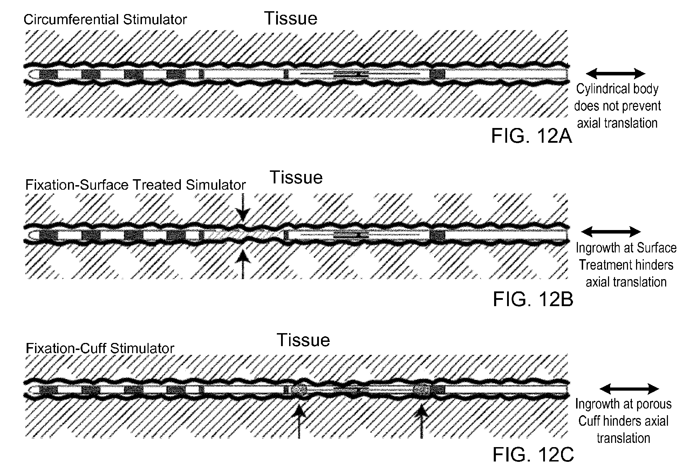

FIG. 12A illustrates an implementation of a cylindrical stimulator device without tissue-ingrowth promoting features while implanted in tissue. As shown, the materials and shape of the housing (cylindrical) do not necessarily prevent axial translation of the device.

FIG. 12B illustrates an implementation of a cylindrical stimulator device with tissue-ingrowth surface treatment while implanted in tissue. Tissue ingrowth at the surface treatment hinders axial translation of the device.

FIG. 12C illustrates an implementation of a cylindrical stimulator device with tissue-ingrowth cuff while implanted in tissue. Tissue ingrowth at the porous cuff hinders axial translation of the device.

A number of implementations have been described. Nevertheless, it will be understood that various modifications may be made. Accordingly, other implementations are within the scope of the following claims.

## Claims

1. A wireless neural stimulator device comprising:
a housing configured to house:
a first antenna configured to receive, from a second antenna and through electrical radiative coupling, an input signal containing electrical energy, the second antenna being physically separate from the wireless neural stimulator device;
one or more flexible circuits electrically connected to the first antenna, the flexible circuits configured to create the one or more electrical pulses suitable to be applied at the electrodes using the electrical energy contained in the input signal and supply the one or more electrical pulses to one or more electrodes;
one or more electrodes disposed on the housing, the one or more electrodes being coupled to the flexible circuits to receive the one or more electrical pulses supplied by the one or more flexible circuits
one or more fixation features disposed on the housing and configured to fixate the device to tissue.

2. The device of claim 1 wherein the one or more fixation features include a cuff formed from a porous material that promotes tissue in-growth.

3. The device of claim 1 wherein the one or more fixation features include a surface feature configured to promote tissue adhesion

4. The device of claim 3 wherein the surface feature includes an altered surface area that is increased relative to the surface area of other portions of the housing.

5. The device of claim 3 wherein the surface feature includes spirals or dimples.

6. The device of claim 1 wherein the one or more fixation features include a tine band.

7. The device of claim 6 wherein the tine band includes a hub with a central opening and one or more tines extending radially from the hub and forming an acute angle with respect to a central axis passing through a center of the opening of the hub.

8. The device of claim 7 wherein the tines are resilient.

9. The device of claim 1 wherein the electrodes are spaced from the first antenna such that, when the device is implanted in a patient in a position to stimulate a dorsal root ganglion or exiting nerves of the spinal cord, the first antenna is surrounded by fatty tissue.

10. The device of claim 1 wherein the electrodes and the first antenna are spaced such that, when the device is implanted in a patient in a position to stimulate the sacral nerve, the first antenna is substantially parallel to a surface of a back of the patient.

11. The device of claim 1 wherein the electrodes are configured for monopolar stimulation.

12. The device of claim 11 wherein the electrodes include multiple electrodes disposed at a distal end of the housing and a remote electrode disposed at the proximal end of the housing, the remote electrode configured as an anode for monopolar stimulation.
